(19) Europäisches Patentamt
European Patent Office
Office européen des brevets

(11) Veröffentlichungsnummer: **0 155 518**
**B1**

(12) # EUROPÄISCHE PATENTSCHRIFT

(45) Veröffentlichungstag der Patentschrift: **30.08.89**

(51) Int. Cl.$^4$: **C 07 C127/19**, A 61 K 31/17

(21) Anmeldenummer: **85101802.8**

(22) Anmeldetag: **20.02.85**

(54) **S(-)-Celiprolol, dessen pharmazeutisch verträgliche Salze, Verfahren zu deren Herstellung und pharmazeutische Zubereitung.**

(30) Priorität: **21.03.84 DE 3410380**

(43) Veröffentlichungstag der Anmeldung: **25.09.85 Patentblatt 85/39**

(45) Bekanntmachung des Hinweises auf die Patenterteilung: **30.08.89 Patentblatt 89/35**

(84) Benannte Vertragsstaaten: **AT BE CH DE FR GB IT LI LU NL SE**

(56) Entgegenhaltungen:
**DE–A– 2 458 624**
**JOURNAL OF CHROMATOGRAPHY, Band 316, 21. Dezember 1984, Seiten 605-616, Elsevier Science Publishers B.V., Amsterdam, NL; W. LINDNER et al.: "Liquid chromatographic separation of enantiomeric alkanolamines via diastereomeric tartaric acid monoesters"**
**Die Akte enthält technische Angaben, die nach dem Eingang der Anmeldung eingereicht wurden und die nicht in dieser Patentschrift enthalten sind.**

(73) Patentinhaber: **RORER INTERNATIONAL (OVERSEAS) INC.**
**1209 Orange Street**
**Wilmington Delaware 19899 (US)**

(72) Erfinder: **Zölss, Gerhard, Dr.**
**Pyhrnstrasse 499**
**A-4580 Windischgarsten (AT)**
Erfinder: **Gratz, Richard**
**Peuerbachstrasse 1**
**A-4020 Linz (AT)**
Erfinder: **Schlögl, Karl, Dr.**
**Grünentorgasse 19**
**A-1090 Wien (AT)**
Erfinder: **Gordon, Robert Jay**
**85 Castle Road**
**Chappaqua New York 10514 (US)**
Erfinder: **Hofer, Otmar, Dr.**
**Fuchsthallergasse 12/30**
**A-1090 Wien (AT)**

(74) Vertreter: **Müllner, Erwin, Dr. et al**
**Patentanwälte Dr. Erwin Müllner Dipl.-Ing. Werner Katschinka Postfach 159 Weihburggasse 9**
**A-1010 Wien (AT)**

**EP 0 155 518 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft den S(—)-3-[3-Acetyl-4-(3-tert.butylamino-2-hydroxypropoxy)phenyl]-1,1-diäthylharnstoff, (S(—)-Celiprolol), dessen pharmazeutisch verträgliche Salze, Verfahren zu dessen Herstellung sowie pharmazeutische Zubereitungen, die S(—)-Celiprolol als Wirkstoff enthalten.

Die DE-PS-2,458.624 beschreibt unter anderem das Racemat des 3-[3-Acetyl-4-(3-tert.butylamino-2-hyroxy)propoxyphenyl]-1,1-diäthylharnstoffs (Celiprolol) mit der nachfolgenden Formel

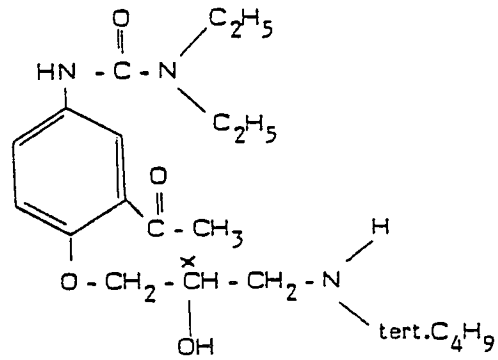

(I)

als Substanz mit $beta_1$-Rezeptoren blockierender Wirkung.

Beta-Blocker sind Substanzen mit wertvollen pharmakologischen Eigenschaften und haben große Bedeutung bei der Behandlung von Erkrankungen des Herzens und des Kreislaufs wie Angina pectoris, Myokardinfarkt, Herzrhythmusstörungen oder Bluthochdruck erlangt.

Aus der DE-PS-2,458.624 ist auch bekannt, daß Celiprolol eine ausgesprochen cardioselektive $beta_1$-blockierende Wirkung besitzt, während die sonst bei Beta-Blockern üblichen cardiodepressiven Nebenwirkungen nicht beobachtet werden.

In der Struktur der Verbindung der Formel I ist das mit einem Stern gekennzeichnete Kohlenstoffatom in der basischen Seitenkette ein Chiralitätszentrum, sodaß Celiprolol in zwei optischen Antipoden vorliegen kann.

Aus der EP-A-15418 oder Nature, 210, 1336 ff ist weiters auch bekannt, daß bei Beta-Blockern wie Moprolol (1-Isopropylamino-3-(o-methoxy-phenoxy)-2-propanol) oder Propranolol (1-Isopropylamino-3-(1-naphthoxy)-2-propanol) die $beta_1$-Rezeptoren blockierende Wirkung hauptsächlich dem linksdrehenden Enantiomeren der genannten Verbindungen zuzuschreiben ist, während das rechtsdrehende Enantiomere nur geringe oder überhaupt keine $beta_1$-Rezeptoren blockierende Wirkung besitzt. Andere vorteilhafte pharmakologische Eigenschaften sind bisher für die linksdrehenden Formen von chiralen Beta-Blockern nicht bekannt geworden.

Es wurde nun gefunden, daß das linksdrehende Enantiomere von Celiprolol nicht nur eine gegenüber dem Racemat stärkere $beta_1$-Rezeptoren blockierende Wirkung sondern überraschenderweise auch eine deutlich ausgeprägte bronchialerweiternde Wirkung besitzt.

Gegenstand der vorliegenden Erfindung sind demnach S(—)-3-[3-Acetyl-4-(3-tert.butylamino-2-hydroxy)propoxyphenyl]-1,1-diäthylharnstoff (S(—)-Celiprolol) und dessen pharmazeutisch verträglichen Additionssalze mit Säuren.

Zur Herstellung des linksdrehenden Celiprolol mit der S-Konfiguration kommen zwei an sich bekannte Methoden in Betracht:

a) Im Verlaufe einer Racematspaltung kann S(—)-Celiprolol durch Umsetzung des Racemats der Formel I mit einem Enantiomeren einer chiralen Spaltsäure, Auftrennung des bei der Umsetzung gebildeten Gemisches der beiden diastereomeren Salze und erneute Freisetzung der Base aus dem Salz in guten Ausbeuten gewonnen werden.

Als chirale Spaltsäuren, die zur Bildung des Diastereomerengemisches in einer der beiden optisch aktiven Formen vorliegen müssen, eignen sich die für solche Zwecke üblichen Säuren wie beispielsweise Weinsäure und deren Derivate, Mandelsäure, Campher-beta-sulfonsäure oder Chinasäure. Wegen der guten Kristallisationstendenz des jeweils aus der Reaktionslösung bevorzugt auskristallisierenden diastereomeren Salzes und der damit verbundenen leichten Auftrennbarkeit des diastereomeren Salzgemisches eignen sich besonders Derivate der D- oder L-Weinsäure für die erfindungsgemäße Racemattrennung. Vorteilhafterweise setzt man dabei racemisches Celiprolol mit jeweils einem Enantiomeren der Di-0,0'-p-toluoyl-weinsäure oder der Di-0,0'-benzoylweinsäure in Form ihrer Hydrogentartrate um. Besonders bevorzugt verwendet man zur Racematspaltung (+)-Di-0,0'-benzoyl-D-weinsäure oder (+)-Di-0,0'-p-toluoyl-D-weinsäure. Ganz besonders bevorzugt erfolgt die Umsetzung mit (+)-Di-0,0'-benzoyl-D-weinsäure.

Bei der Durchführung des Verfahrens geht man zweckmäßigerweise so vor, daß man racemisches Celiprolol in Form der freien Base in einem geeigneten Lösungsmittel mit der Spaltsäure umsetzt und das

Reaktionsgemisch gegebenenfalls gelinde, beispielsweise auf eine Temperatur bis höchstens 50 °C, vorzugsweise bis höchstens 30 °C erwärmt, bis eine klare rückstandsfreie Lösung entsteht. Die Racemattrennung erfolgt dann durch fraktioniertes Aus- und Umkristallisieren eines der gebildeten diastereomeren Salze und Abtrennen derselben aus der Mutterlauge, wobei je nach Art der eingesetzten Spaltsäure und des Lösungsmittels entweder das Additionssalz von S(—)-Celiprolol oder von R(+)-Celiprolol mit dem Enantiomeren der Spaltsäure bevorzugt aus der Lösung auskristallisiert und von dem in der Mutterlauge verbliebenen Diastereomeren abgetrennt werden kann.

Geeignete Lösungsmittel für die Umsetzung mit der Spaltsäure und für die Racematspaltung sind polare Lösungsmittel wie Wasser, Alkohole, Ketone, beispielsweise Aceton, Acetonitril oder Gemische dieser Lösungsmittel mit Wasser, weiters Chloroform oder Essigester.

Bevorzugte Lösungsmittel sind niedere aliphatische Alkohole, wobei wiederum die Verwendung von Methanol oder Äthanol und deren Gemische mit Wasser besonders bevorzugt ist.

Die fraktionierte Kristallisation wird vorteilhafterweise durch langsames Abkühlen der Reaktionslösung auf Temperaturen unterhalb der Raumtemperatur bis —10 °C und/oder Zusatz eines Impfkristalles eingeleitet und durch mehrstündiges bis mehrtägiges Stehen in der Kälte vollendet.

In einer besonders günstigen Ausführungsform des Verfahrens wird die Racemattrennung so ausgeführt, daß bei der fraktionierten Kristallisation jenes der beiden diastereomeren Salze, welches als Base S(—)-Celiprolol enthält, bevorzugt aus der Lösung auskristallisiert und abgetrennt werden kann. Man kann das zweckmäßigerweise dadurch erreichen, indem man das Racemat beispielsweise mit (+)-Di-0,0′-p-toluoyl-D-weinsäure oder (+)-Di-0,0′-benzoyl-D-weinsäure als Spaltmittel in Methanol umsetzt. Dabei fällt das S-Celiprolol-Di-0,0′-p-toluoyl-D-tartrat oder S-Celiprolol-Di-0,0′-benzoyl-D-tartrat bei Abkühlung auf Temperaturen unterhalb der Raumtemperatur in großflächigen Kristallen nahezu quantitativ aus und kann von dem in der Mutterlauge verbleibenden diastereomeren Salz des (R)-Celiprolols auf übliche Weise wie durch Filtrieren, Zentrifugieren oder Dekantieren leicht abgetrennt und bis zur Erreichung eines konstanten Schmelzpunktes und konstanter optischer Drehung umkristallisiert werden. Aus diesen Salzen wird die linksdrehende Celiprolol-Base mit der S-Konfiguration durch Zugabe von basischen Substanzen wie z. B. Natrium- oder Kaliumhydroxyd unter Rückgewinnung der Spaltsäure wieder freigesetzt und kann dann gewünschtenfalls durch Behandeln mit Säuren, beispielsweise mit Mineralsäuren, wie Chlorwasserstoffsäure, Bromwasserstoffsäure, Schwefelsäure oder Phosphorsäure in ein pharmazeutisch verträgliches Additionssalz übergeführt werden.

Additionssalze von S(—)-Celiprolol mit Mineralsäuren können in ganz besonders bevorzugter Weise ohne Freisetzung der Base auch dann erhalten werden, wenn man die nach der Racemattrennung erhaltenen Additionssalze aus S(—)-Celiprolol und der Spaltsäure, beispielsweise S(—)-Celiprolol-Di-p-toluoyl-D-hydrogentartrat oder S(—)-Celiprolol-Dibenzoyl-D-hydrogentartrat, direkt mit der gewünschten Mineralsäure, beispielsweise Salzsäure, in einem geeigneten Lösungsmittel umsetzt, in dem die freigesetzte organische Spaltsäure gut löslich ist, während das entsprechende Mineralsalz von S(—)-Celiprolol schwer löslich ist und ausfällt. Geeignete Lösungsmittel für diesen Zweck sind beispielsweise Ketone wie Aceton, Methyläthylketon, Acetonitril, Chloroform oder Essigester, wobei die Verwendung von Aceton ganz besonders bevorzugt ist.

Eine Reindarstellung von S(—)-Celiprolol ist aber auch dann möglich, wenn durch die Wahl einer entsprechenden Spaltsäure jenes der beiden diastereomeren Salze, welches R(+)-Celiprolol als Base enthält, bei der fraktionierten Kristallisation ausfällt und abgetrennt wird. In diesem Fall wird die linksdrehende Celiprolol-Base mit der S-Konfiguration in der Mutterlauge aus dem zur Racemattrennung hergestellten Additionssalz unter Rückgewinnung der Spaltsäure wieder freigesetzt und dann beispielsweise durch Extraktion und Umkristallisieren in reiner Form gewonnen. Bei der Durchführung des Verfahrens kann man beispielsweise so vorgehen, daß man aus dem nach der Racemattrennung in der Mutterlauge anfallenden diastereomeren Salz die organische Spaltsäure durch Behandeln mit Mineralsäuren wie Chlorwasserstoffsäure, Bromwasserstoffsäure, Schwefelsäure oder Phosphorsäure freisetzt und aus dem Reaktionsgemisch extrahiert. Aus dem dabei gebildeten Mineralsalz des S(—)-Celiprolol kann wiederum das basische S(—)-Celiprolol durch Einwirkung starker Basen, wie beispielsweise Natronlauge, freigesetzt und in eine organische Phase extrahiert werden. Die freie Base kann dann gewünschtenfalls in ein pharmazeutisch verträgliches Säureadditionssalz übergeführt werden.

S(—)-Celiprolol kann aber auch

b) durch Umsetzung von S(+)-3-[3-Acetyl-4-(2,3-epoxy-propoxy)-phenyl]-1,1-diäthylharnstoff der Formel

(Siehe Schema Seite 4 f.)

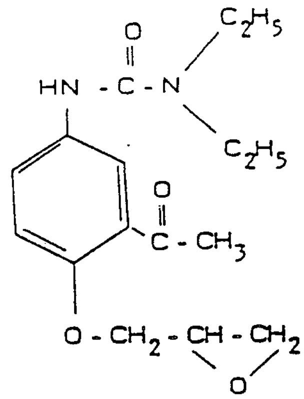

(II)

mit tert. Butylamin in Gegenwart von Wasser erhalten werden.

Solche Umsetzungen von ähnlichen enantiomeren Epoxyden mit Aminen, bei denen unter Erhaltung der Konfiguration am Chiralitätszentrum die entsprechenden optisch aktiven Aminoalkohole entstehen, sind aus der Literatur bekannt, z. B. W.L. Nelson, J.E.Wennerstrom and S.R.Sankar, J.Org.Chem. Vol.42, N°.6, 1977, Seiten 1006 - 1012.

Bei der Durchführung der Reaktion geht man zweckmäßigerweise so vor, daß man das tert. Butylamin gemischt mit Wasser, beispielsweise in etwa gleichen Mengen, vorlegt, das Epoxyd der Formel II mit der S-Konfiguration langsam einträgt und bei Raumtemperatur oder unter gelindem Erwärmen auf höchstens 40 °C vorzugsweise höchstens 30 °C miteinander zur Reaktion bringt. Das dabei erhaltene linksdrehende Enantiomere des Celiprolols kann dann wiederum durch Behandeln mit Säuren in ein pharmazeutisch verträgliches Additionssalz übergeführt werden. Das bei dieser Umsetzung erhaltene Produkt ist mit dem durch Racemattrennung hergestellten S(—)-Celiprolol hinsichtlich der chemischen und physikalischen Eigenschaften identisch. Insbesondere erhält man nach den beiden oben angeführten Herstellungsmethoden Produkte, welche denselben Drehwert, einen identen Kurvenverlauf im CD-Spektrum und identische NMR-Spektren mit oder ohne Verschiebungsreagenzien aufweisen.

Von dem als Ausgangsmaterial eingesetzten enantiomeren S(+)-3-[3-Acetyl-4-(2,3-epoxy-propoxy)-phenyl]-1,1-diäthylharnstoff der Formel II ist bisher nur das Racemat in der DE-PS-2,458.624 beschrieben. Die für die Herstellung von S(—)-Celiprolol benötigte rechtsdehende Form des 3-[3-Acetyl-4-(2,3-epoxypropoxy)-phenyl]-1,1-diäthylharnstoffs mit der S-Konfiguration ist beispielsweise nach der von W.L. Nelson, J.E.Wennerstrom und S.R. Sankar im J. Org. Chem. Vol 42, N°. 6, 1977, Seiten 1006 - 1012 angegebenen Reaktionsfolge aus S(+)-2,2-Dimethyl-4-(p-tosyloxy-methyl)-1,3-dioxolan und 3-(3-Acetyl-4-hydroxy-phenyl)-1,1-diäthylharnstoff oder nach der von G. Zölß in Drug. Res. 33,2 (1983) angegebenen Methode durch Umsetzung von 3-(3-Acetyl-4-hydroxy-phenyl)-1,1-diäthylharnstoff mit R(—)-Epichlorhydrin gut zugänglich. Der Drehwert von S(+)-3-[3-Acetyl-4-(2,3-epoxy-propoxy)-phenyl]-1,1-diäthylharnstoff wurde bestimmt zu $[alpha]^{22}_{589}$ : + 12.6° (c = 2,8 in Aceton).

Die in dieser Beschreibung und den Patentansprüchen zur Bezeichnung der absoluten Konfiguration verwendete Bezeichnung R (rectum) und S (sinister) richtet sich nach dem Artikel in « Experientia », Band 12, Seiten 81 - 94 (1956).

Die pharmakologische Untersuchung der erfindungsgemäßen Verbindung zeigt, daß S(—)-Celiprolol neben der ausgeprägt cardioselektiven $beta_1$-Rezeptorenblockierenden Wirkung überraschenderweise auch eine deutliche und anhaltende bronchodilatorische Wirkung besitzt. Diese bronchodilatorische Wirkung kann beispielsweise im Tierversuch an anästhesierten Katzen, deren Bronchialtonus durch eine konstante intravenöse Infusion von Serotonin erhöht worden ist, nachgewiesen werden.

Dieses Ergebnis ist deshalb überraschend und unerwartet, da bekannt ist, daß Beta-Blocker generell zu einer Zunahme des Atemwegswiderstandes führen und deshalb für Patienten, die unter Asthma bronchiale oder anderen obstruktiven Atemwegserkrankungen leiden, kontraindiziert sind.

Bei Celiprolol dagegen wird durch die linksdrehende Form mit der S-Konfiguration in allen untersuchten Fällen eine deutliche und anhaltende bronchialerweiternde Wirkung ausgelöst, während bei der Verabreichung der rechtsdrehenden Form mit der R-Konfiguration im Tierversuch nach einer anfänglichen kurzzeitigen Bronchodilatation der Brochialtonus wieder auf den ursprünglichen Wert ansteigt und in etwa einem Viertel der untersuchten Fälle sogar eine beachtliche bronchokonstriktorische Wirkung auftritt.

Aufgrund dieser überraschenden pharmakologischen Eigenschaften kann S(—)-Celiprolol in der Therapie zur Behandlung von Erkrankungen des Herzens und des Kreislaufs ohne Risiko insbesondere auch bei solchen Patienten verwendet werden, bei denen die Gabe von Beta-Blockern wegen obstruktiver Atemwegserkrankungen bisher kontraindiziert war, insbesondere zur Therapie des Bluthochdrucks, der Angina pectoris, tachykarder Herzrythmusstörungen und ähnlicher Erkrankungen, die mit einer unerwünscht hohen Belastung des Kreislaufs mit körpereigenen Catecholaminen erklärt werden können.

Gegenstand der Erfindung sind auch pharmazeutische Zubereitungen, die S(—)-Celiprolol oder dessen pharmazeutisch verträglichen Salze als Wirkstoff in Kombination mit pharmakologisch annehmbaren Bindemitteln, Trägermaterialien und/oder Hilfsstoffen enthalten. Die erfindungsgemäßen pharmazeutischen Zubereitungen können, wie bei Beta-Blockern üblich, auf oralem oder parenteralem Weg an den Patienten verabreicht werden.

Die nachfolgenden Beispiele dienen der weiteren Erläuterung der Erfindung, namentlich der oben beschriebenen Methoden zur Herstellung von S(—)-Celiprolol.

Beispiel 1

100 g racemische Celiprolol-Base werden in 600 ml EtOH 96 %ig gelöst und mit 106,6 g (+)-Di-0,0'-p-toluoyl-D-weinsäure versetzt. Nach gelindem Erwärmen auf 28 °C wird eine klare Lösung erhalten. Diese wird angeimpft und zur Kristallisation 3 Tage auf —3 °C abgekühlt. Das ausgeschiedene Salz wird abgesaugt, mit EtOH gewaschen und an der Luft getrocknet. Man erhält so eine Ausbeute von 122,6 g an S-Celiprolol-Di-0,0'-p-toluoyl-D-tartrat.

120 g dieses diastereomeren Salzes werden mit 1 200 ml Wasser versetzt, 400 ml Diethylether und anschließend 76,5 ml 4n HCl zugefügt und 20 Minuten bei Raumtemperatur gerührt und die Schichten getrennt. Aus der organischen Phase kann die Spaltsäure wiedergewonnen werden.

Die wässerige Lösung wird mit 200 ml Chloroform versetzt und mit 76,5 ml 4n NaOH alkalisiert. Nach gründlicher Durchmischung werden die Schichten getrennt und die wässerige Phase noch 3 mal mit je 100 ml Chloroform extrahiert. Die Chloroformauszüge werden vereinigt, mit $Na_2SO_4$ sicc. getrocknet und das Lösungsmittel bei 30 °C Badtemperatur entfernt.

Der Eindampfrest wird mit 100 ml Diethylether 30 Minuten digeriert und das ausgefallene Kristallisat abgesaugt. Die Mutterlauge wird eingedampft, der Rückstand in 40 ml Diethylether gelöst und über Nacht bei —20 °C zur Kristallisation gebracht. Das Kristallisat wird abgesaugt, mit kaltem Diethylether gewaschen und getrocknet.

Ausbeute an S(—)-Celiprolol-Base : 16,45 g

$[alpha]^{22}_{589}$ : — 6,3° (c = 10 % in $CHCl_3$)
CD-Spektren :
CD (in Äthanol) : Delta $epsilon_{320}$ : + 0,08
$Deltaepsilon_{255}$ : — 0,25

Herstellung des Hydrochlorides

12,0 g S(—)-Celiprolol-Base werden in 54 ml Aceton bei 20 °C gelöst, mit der berechneten Menge 4n HCl versetzt und das entstandene Kristallisat nach 2 Stunden bei 4 °C abgesaugt, mit Aceton nachgewaschen und getrocknet.

Ausbeute : 11,2 g S(—)-Celiprolol-hydrochlorid
$[alpha]^{25}_{589}$ : — 7,4° (c = 10 % in Methanol)
MFp. : 187°-188°

Analyse : gef.

| | |
|---|---|
| C = 57,5 % | ber. C = 57,7 % |
| H = 8,5 % | H = 8,24 % |
| N = 9,9 % | N = 10,1 % |
| O = 15,6 % | O = 15,4 % |
| Cl = 8,5 % | Cl = 8,5 % |

Beispiel 2

100,0 g racemische Celiprolol-Base werden in 600 ml 75 Gew.% wässerigem EtOH gelöst, 106,6 g (—)-Di-0,0'-p-toluoyl-L-weinsäure zugefügt und bis zur klaren Lösung erwärmt (28 °C). Nach dem Animpfen wird 3 Tage bei — 3 °C zur Kristallisation gebracht. Das ausgeschiedene Salz wird abgesaugt und nachgewaschen.

Aus der Mutterlauge und der Waschlösung des abgeschiedenen Kristallisates wird der Alkohol i.v. bei 30 °C abdestilliert. Der Rückstand wird durch Zugabe von 500 ml Wasser, 68 ml 4n HCl und 500 ml Diethylether durch Rühren bei Raumtemperatur gelöst, die organische Schichte abgetrennt und die wässerige Lösung noch 5 mal mit Diethylether extrahiert. Aus der etherischen Lösung kann die Spaltsäure auf übliche Weise rückgewonnen werden.

Die wässerige Lösung wird mit 68 ml 4n NaOH alkalisiert und die ausgeschiedene Base mehrmals mit Chloroform extrahiert. Die vereinigten Extrakte werden über $Na_2SO_4$ sicc. getrocknet und das Lösungsmittel i.v. bei 30 °C entfernt. Der Rückstand wird mit 100 ml Diethylether 30 Minuten digeriert, das entstandene Kristallisat abgetrennt und die Mutterlauge bei 30 °C eingedampft. Der Rückstand wird in 40 ml Diethylether aufgenommen und über Nacht bei — 20 °C zur Kristallisation gebracht.

Ausbeute an S(—)-Celiprolol-Base : 12,2 g
$[alpha]^{23}_{589}$ : — 6,4° (c = 10 % in $CHCl_3$)

Beispiel 3

18,9 g racemische Celiprolol-Base werden in 100 ml Methanol gelöst und mit einer warmen Lösung von 19,3 g (—)-0,0'-Dibenzoyl-L-weinsäure-hydrat in 150 ml Methanol versetzt. Die Lösung wird in der Wärme noch mit 110 ml Methanol und 360 ml Wasser versetzt, langsam auf Raumtemperatur abgekühlt und 20 Stunden bei 5 °C bis 8 °C gehalten.

Das ausgefallene Kristallisat wird abgesaugt und mit 50 ml Methanol/Wasser 1 : 1 gewaschen.

Die Waschlösung und die Mutterlauge werden i.v. auf cas. 400 ml eingeengt, mit 20 g NaOH in 30 ml Wasser versetzt und mit Methylenchlorid ausgeschüttelt. Die organische Phase wird mit verd. NaOH und Wasser gewaschen, getrocknet und i.v. eingedampft. Der Kristalline Rückstand wird mit 300 ml Diethylether erwärmt, Unlösliches abgetrennt und die Mutterlauge mit Petrolether zur Kristallisation gebracht.

Ausbeute : 5,15 g
$[alpha]^{22}_{589}$ : — 6,5° ($CHCl_3$)

Beispiel 4

198,5 g racemische Celiprolol-Base werden in 1 000 ml Methanol bei 40 °C gelöst und mit einer Lösung von 193,05 g (+)-0,0'-Dibenzoyl-D-weinsäurehydrat in 1 500 ml Methanol bei 40 °C versetzt, weitere 1 100 ml Methanol zugegeben und mit 3 600 ml Wasser von 40 °C versetzt.

Bei etwa 30 °C wird angeimpft, 2 Stunden bei Raumtemperatur und 24 Stunden bei 4 °C zur Vervollständigung der Kristallisation stehen gelassen. Das ausgefallene Kristallisat wird abgesaugt und getrocknet. Man erhält so eine Ausbeute von 180 g.

179 g dieser Substanz werden aus 3 570 ml Methanol-Wasser (1 : 1) umkristallisiert. Nach 7 Stunden bei 3 °C - 4 °C wird das Kristallisat abgesaugt und getrocknet.

Die Ausbeute an S(—)-Celiprolol-0,0'-dibenzoyl-D-hydrogentartrat beträgt 149,5 g.

$[alpha]^{23}_{589}$ : + 54,6° (c = 10 % in MeOH)
MF p. : 143-145 °C

Umwandlung in S(—)-Celiprolol-hydrochlorid :

142 g des so erhaltenen S(—)-Celiprolol-0,0'-dibenzoyl-D-hydrogentartrates werden in 2130 ml Aceton gelöst und unter Rühren mit 16,5 ml konz HCl (entsprechend 7,08 g HCl) versetzt. Anschließend wird 2 Stunden mit Eiswasser gekühlt und das entstandene S(—)-Celiprolol-hydrochlorid abgesaugt und mehrmals mit Aceton gewaschen.

Ausbeute : 77,0 g
$[alpha]^{25}_{589}$ : — 7,9° (c = 10 % in MeOH)

Beispiel 5

In eine Mischung von 0,86 ml tert.-Butylamin und 0,86 ml Wasser werden 250 mg S(+)-3-[3-Acetyl-4-(2,3-epoxy-propoxy)-phenyl]-1,1-diethylharnstoff bei Raumtemperatur eingetragen und 6 Stunden bei Raumtemperatur gerührt. Das überschüssige tert.-Butylamin wird sodann ohne Erwärmung im Vakuum abdestilliert. Der Rückstand wird mit Wasser verdünnt und die ausgeschiedene Base erschöpfend mit Chloroform extrahiert. Die Chloroformlösung wird nach dem Trocknen mit $Na_2SO_4$ sicc. im Vakuum vollständig eingeengt.

Der erhaltene Rückstand wird mit 1,5 ml Aceton versetzt, angeimpft und zur Vervollständigung der Kristallisation 5 Stunden auf 0 °C abgekühlt, das ausgefallene Kristallisat abgesaugt und getrocknet.

Ausbeute an S(—)-Celiprolol : 225 mg
$[alpha]^{22}_{589}$ : — 6,3° ($CHCl_3$)

**Patentansprüche** (für die Vertragsstaaten : BE, CH, DE, FR, GB, IT, LI, LU, NL, SE)

1. S(—)-3-[3-Acetyl-4-(3-tert.butylamino-2-hydroxypropoxy)phenyl]-1,1-diäthylharnstoff (S(—)-Celiprolol) und dessen pharmazeutisch verträgliche Additionssalze mit Säuren.

2. Verfahren zur Herstellung der Verbindung gemäß Anspruch 1 und von deren Salzen dadurch gekennzeichnet, daß man

a) das Racemat der Formel

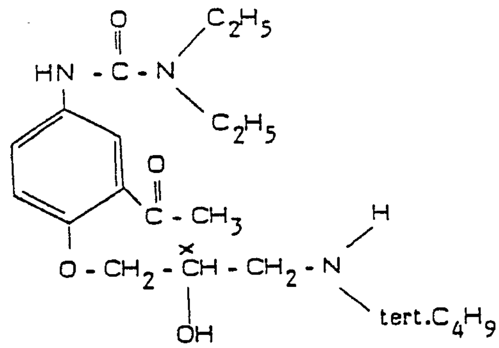

(I)

in Form der Base mit einem Enantiomeren einer chiralen Spaltsäure in einem geeigneten organischen Lösungsmittel umsetzt, das auf diese Weise gebildete Gemisch der beiden diastereomeren Salze durch fraktionierte Kristallisation auftrennt und nach der Trennung jenes diastereomere Salz, welches das linksdrehende Enantiomere der Verbindung der Formel I in der S-Konfiguration enthält, wieder in die freie Base oder ein pharmazeutisch verträgliches Additionssalz überführt und die Spaltsäure zurückgewinnt, oder

b) S(+)-3-[3-Acetyl-4-(2,3-epoxy-propoxy)-phenyl]-1,1-diäthylharnstoff der Formel

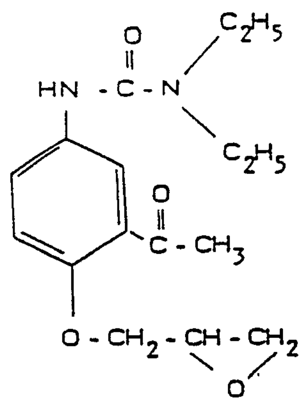

(II)

mit tert. Butylamin in Gegenwart von Wasser bei Raumtemperatur oder unter gelindem Erwärmen auf maximal 40 °C umsetzt und dann das dabei erhaltene linksdrehende Enantiomere der Verbindung der Formel I gewünschtenfalls in ein pharmazeutisch verträgliches Additionssalz überführt.

3. Verfahren nach Anspruch 2, dadurch gekennzeichnet, daß man bei der Racemattrennung als chirale Spaltsäure ein Enantiomeres der Di-0,0'-p-toluoyl- oder Di-0,0'-benzoylweinsäure einsetzt.

4. Verfahren nach Anspruch 2, dadurch gekennzeichnet, daß man das Racemat der Formel I mit (+)-Di-0,0'-p-toluoyl-D-weinsäure oder (+)-Di-0,0'-benzoyl-D-weinsäure als Spaltsäure umsetzt und das bei der fraktionierten Kristallisation ausfallende S-Celiprolol-Di-0,0'-p-toluoyl-D-tartrat oder S-Celiprolol-Di-0,0'-benzoyl-D-tartrat aus der Mutterlauge abtrennt.

5. Verfahren nach den Ansprüchen 2 bis 4, dadurch gekennzeichnet, daß man bei der Racemattrennung die Salzbildung mit der chiralen Säure und die fraktionierte Kristallisation in Methanol, Äthanol oder einem Gemisch eines dieser Alkohole mit Wasser durchführt.

6. Verfahren nach den Ansprüchen 2 bis 5, dadurch gekennzeichnet, daß man das nach der Racemattrennung erhaltene diastereomere Salz aus S(—)-Celiprolol und Spaltsäure direkt mit Mineralsäuren zu pharmazeutisch verträglichen Additionssalzen in einem Lösungsmittel umsetzt, in dem die freigeseizte organische Spaltsäure gut löslich und das bei der Umsetzung gebildete Mineralsalz von S(—)-Celiprolol schwer löslich ist und ausfällt.

7. Verfahren nach den Ansprüchen 2 bis 6, dadurch gekennzeichnet, daß man zur Herstellung der pharmazeutisch verträglichen Additionssalze Salzsäure verwendet.

8. Verfahren nach den Ansprüchen 2 bis 7, dadurch gekennzeichnet, daß man die direkte Umsetzung der nach der Racemattrennung erhaltenen Salze aus S(—)-Celiprolol und Spaltsäure mit Mineralsäuren in Aceton als Lösunsmittel durchführt.

9. Pharmazeutische Zubereitung, dadurch gekennzeichnet, daß sie aus der Verbindung gemäß Anspruch 1 oder deren Salzen und üblichen pharmakologisch annehmbaren Bindemitteln, Trägermaterialien und/oder Hilfsstoffen besteht.

**Patentansprüche** (für den Vertragsstaat AT)

1. Verfahren zur Herstellung von S(—)-3-[3-Acetyl-4-(3-tert.butylamino-2-hydroxypropoxy)-phenyl]-1,1-diäthylharnstoff (S(—)-Celiprolol) und dessen pharmazeutisch verträglichen Additionssalzen mit Säuren, dadurch gekennzeichnet, daß man

a) das Racemat der Formel

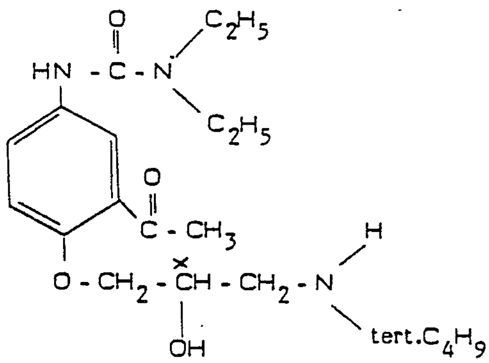

(I)

in Form der Base mit einem Enantiomeren einer chiralen Spaltsäure in einem geeigneten organischen Lösungsmittel umsetzt, das auf diese Weise gebildete Gemisch der beiden diastereomeren Salze durch fraktionierte Kristallisation auftrennt und nach der Trennung jenes diastereomere Salz, welches das linksdrehende Enantiomere der Verbindung der Formel I in der S-Konfiguration enthält, wieder in die freie Base oder ein pharmazeutisch verträgliches Additionssalz überführt und die Spaltsäure zurückgewinnt, oder

b) S(+)-3-[3-Acetyl-4-(2,3-epoxy-propoxy)-phenyl]-1,1-diäthylharnstoff der Formel

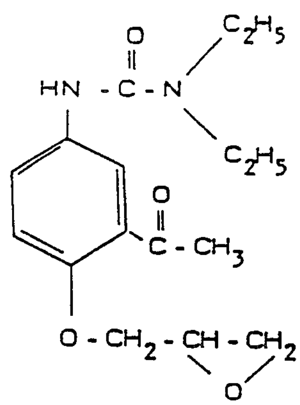

(II)

mit tert. Butylamin in Gegenwart von Wasser bei Raumtemperatur oder unter gelindem Erwärmen bis maximal 40 °C umsetzt und dann das dabei erhaltene linksdrehende Enantiomere der Verbindung der Formel I gewünschtenfalls in ein pharmazeutisch verträgliches Additionssalz überführt.

2. Verfahren nach Anspruch 1, dadurch gekennzeichnet, daß man bei der Racemattrennung als chirale Spaltsäure ein Enantiomeres der Di-0,0'-p-toluoyl-oder Di-0,0'-benzoylweinsäure einsetzt.

3. Verfahren nach den Ansprüchen 1 und 2 dadurch gekennzeichnet, daß man das Racemat der Formel I mit (+)-Di-0,0'-p-toluoyl-D-weinsäure oder (+)-Di-0,0'-benzoyl-D-weinsäure als Spaltsäure umsetzt, wobei das bei der fraktionierten Kristallisation als Niederschlag anfallende S-Celiprolol-Di-0,0'-p-toluoyl-D-tartrat oder S-Celiprolol-Di-0,0'-benzoyl-D-tartrat aus der Mutterlauge abgetrennt wird.

4. Verfahren nach den Ansprüchen 1 bis 3, dadurch gekennzeichnet, daß man bei der Racemattrennung die Salzbildung mit der chiralen Säure und die fraktionierte Kristallisation in Methanol, Äthanol oder einem Gemisch eines dieser Alkohole mit Wasser durchführt.

5. Verfahren nach den Ansprüchen 1 bis 4, dadurch gekennzeichnet, daß man das nach der Racemattrennung erhaltene diastereomere Salz aus S(—)-Celiprolol und Spaltsäure direkt mit Mineralsäuren zu pharmazeutisch verträglichen Additionssalzen in einem Lösungsmittel umsetzt, in dem die freigesetzte organische Spaltsäure gut löslich und das bei der Umsetzung gebildete Mineralsalz von S(—)-Celiprolol schwer löslich ist und ausfällt.

6. Verfahren nach den Ansprüchen 1 bis 5, dadurch gekennzeichnet, daß man zur Herstellung der pharmazeutisch verträglichen Additionssalze Salzsäure verwendet.

7. Verfahren nach den Ansprüchen 1 bis 6, dadurch gekennzeichnet, daß man die direkte Umsetzung der nach der Racemattrennung erhaltenen Salze aus S(—)-Celiprolol und Spaltsäure mit Mineralsäuren in Aceton als Lösungsmittel durchführt.

**Claims** (for the Contracting States : BE, CH, DE, FR, GB, IT, LI, LU, NL, SE)

1. S(—)-3-[3-acetyl-4-(3-tert.butylamino-2-hydroxypropoxy)phenyl]-1,1-diethylurea (S(—)-celiprolol) and its pharmaceutically acceptable acid addition salts.

2. Process for the preparation of the compound according to claim 1 and of its salts, characterised in that

a) the racemate of the formula

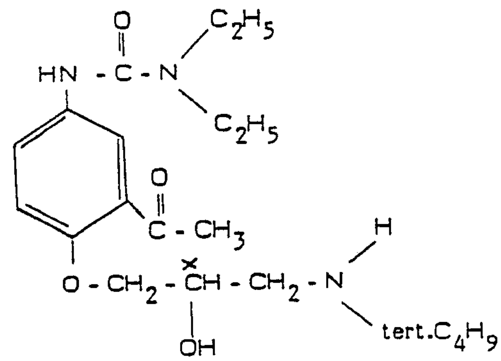

(I)

in the form of the base is reacted with an enantiomer of a chiral resolving acid in a suitable organic solvent, the mixture of the two diastereomeric salts formed in this manner is separated by fractional crystallisation, and, after separating each diastereomeric salt which contains the laevorotatory enantiomer of the compound of the formula I in the S configuration, is reconverted into the free base or a pharmaceutically acceptable addition salt and the resolving acid is recovered, or

b) S(+)-3-[3-acetyl]-4-(2,3-epoxypropoxy)-phenyl]-1,1-diethylurea of the formula

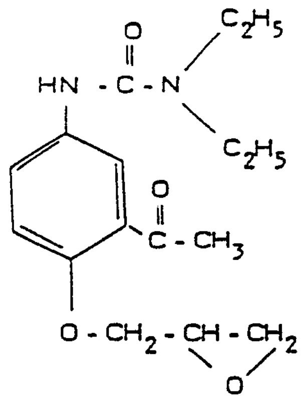

(II)

is reacted with tertiary butylamine in the presence of water at room temperature or with gentle heating to a maximum of 40 °C, and then the laevorotatory enantiomer of the compound of the formula I thus obtained is converted as required into a pharmaceutically acceptable addition salt.

3. Process according to claim 2, characterised in that an enantiomer of di-0,0′-p-toluoyl tartaric acid or di-0,0′-benzoyl tartaric acid is used as the chiral resolving acid for the separation of racemates.

4. Process according to claim 2, characterised in that the racemate of the formula I is reacted with (+)-di-0,0′-p-toluoyl-D tartaric acid or (+)-di-0,0′-benzoyl-D tartaric acid as the resolving acid, and the S-celiprolol-di-0,0′-p-toluoyl-D tartrate or S-celiprolol-di-0,0′-benzoyl-D tartrate precipitated during the fractional crystallisation is removed from the mother liquor.

5. Process according to claims 2 to 4, characterised in that for the separation of racemates, the salt is formed using the chiral acid, and the fractional crystallisation is carried out in methanol, ethanol or a mixture of one of these alcohols with water.

6. Process according to claims 2 to 5, characterised in that the diastereomeric salt of S(—)-celiprolol obtained from the separation of racemates and resolving acid are reacted directly with mineral acids to give pharmaceutically acceptable addition salts in a solvent in which the organic resolving acid released is very soluble, and the mineral salt of S(—)-celiprolol formed in the reaction is difficult to dissolve and is precipitated.

7. Process according to claims 2 to 6, characterised in that hydrochloric acid is used for the preparation of the pharmaceutically acceptable addition salts.

8. Process according to claims 2 to 7, characterised in that the direct reaction of the salts of S(—)-celiprolol obtained from the separation of racemates and resolving acid with mineral acids is carried out in the solvent acetone.

9. Pharmaceutical preparation, characterised in that it consists of the compound according to claim

1 or its salts and conventional pharmacologically acceptable binders, excipients and/or auxiliaries.

**Claims** (for the Contracting State At)

1. Process for the preparation of S(—)-3-[3-acetyl-4-(3-tert.butylamino-2-hydroxypropoxy)phenyl]-1,1-diethylurea (S(—)-celiprolol) and its pharmaceutically acceptable acid addition salts, characterised in that

a) the racemate of the formula

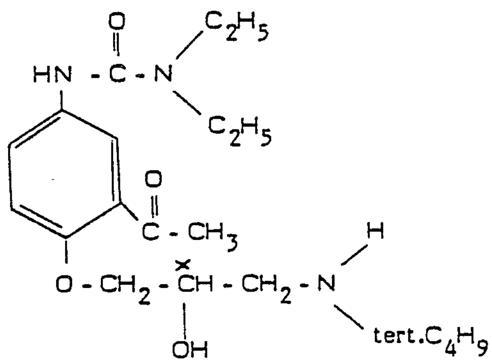

(I)

in the form of the base is reacted with an enantiomer of a chiral resolving acid in a suitable organic solvent, the mixture of the two diastereomeric salts formed in this manner is separated by fractional crystallisation, and, after separating each diastereomeric salt which contains the laevorotatory enantiomer of the compound of the formula I in the S configuration, is reconverted into the free base or a pharmaceutically acceptable addition salt and the resolving acid is recovered, or

b) S(+)-3-[3-acetyl-4-(2,3-epoxypropoxy)-phenyl]-1,1-diethylurea of the formula

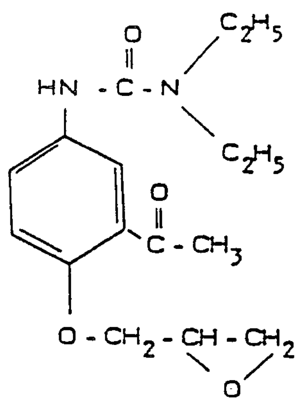

(II)

is reacted with tertiary butylamine in the presence of water at room temperature or with gentle heating to a maximum of 40 °C, and then the laevorotatory enantiomer of the compound of the formula I thus obtained is converted as required into a pharmaceutically acceptable addition salt.

2. Process according to claim 1, characterised in that an enantiomer of di-0,0'-p-toluoyl tartaric acid or di-0,0'-benzoyl tartaric acid is used as the chiral resolving acid for the separation of racemates.

3. Process according to claims 1 and 2, characterised in that the racemate of the formula I is reacted with (+)-di-0,0'-p-toluoyl-D tartaric acid or (+)-di-0,0'-benzoyl-D tartaric acid as the resolving acid, the S-celiprolol-di-0,0'-p-toluoyl-D tartrate or S-celiprolol-di-0,0'-benzoyl-D tartrate occurring as a precipitate during the fractional crystallisation being removed from the mother liquor.

4. Process according to claims 1 to 3, characterised in that for the separation of racemates, the salt is formed using the chiral acid, and the fractional crystallisation is carried out in methanol, ethanol or a mixture of one of these alcohols with water.

5. Process according to claims 1 to 4, characterised in that the diastereomeric salt of S(—)-celiprolol obtained from the separation of racemates and resolving acid are reacted directly with mineral acids to give pharmaceutically acceptable addition salts in a solvent in which the organic resolving acid released is very soluble, and the mineral salt of S(—)-celiprolol formed in the reaction is difficult to dissolve and is precipitated.

6. Process according to claims 1 to 5, characterised in that hydrochloric acid is used for the preparation of the pharmaceutically acceptable addition salts.

7. Process according to claims 1 to 6, characterised in that the direct reaction of the salts of S(—)-celiprolol obtained from the separation of racemates and resolving acid with mineral acids is carried out in the solvent acetone.

**Revendications** (pour les Etats contractants BE, CH, DE, FR, GB, IT, LI, LU, NL, SE)

1. S(—)-3-[3-acétyl-4-(3-tertio-butylamino-2-hydroxypropoxy)phényl]-1,1-diéthylurée (S(—)-céliprolol) et ses sels d'addition d'acide pharmaceutiquement acceptables.

2. Procédé de préparation du dérivé selon la revendication 1 et de ses sels, caractérisé en ce que :

a) l'on fait réagir le racémate de formule

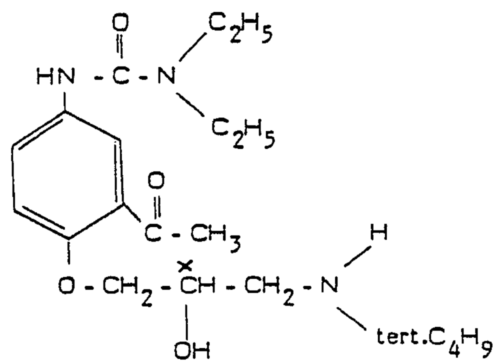

(I)

sous forme de base, avec un énantiomère d'un acide chiral séparable dans un solvant organique approprié, l'on sépare par cristallisation fractionnée le mélange des deux sels diastéréoisomères ainsi obtenu et, après séparation, l'on retransforme chaque sel diastéréoisomère qui contient l'énantiomère lévorotatoire du dérivé de formule I en configuration-S sous forme de base libre ou sous forme d'un sel d'addition d'acide pharmaceutiquement acceptable, et l'on récupère l'acide séparable, ou bien

b) l'on fait réagir la S(+)-3[3-acétyl-4-(2,3-époxy-propoxy)phényl]-1,1-diéthylurée de formule

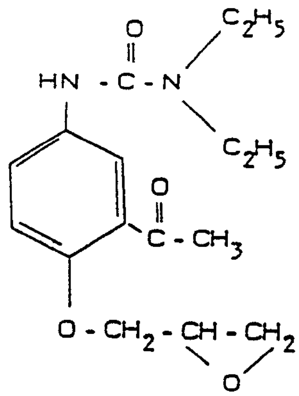

(II)

avec la tertiobutylamine, en présence d'eau, à la température ambiante ou sous chauffage modéré à 40 °C maximum et l'on transforme, le cas échéant, l'énantiomère lévorotatoire de formule I ainsi obtenu en un sel d'addition d'acide pharmaceutiquement acceptable.

3. Procédé selon la revendication 2, caractérisé en ce que, pour la séparation racémique, on emploie comme acide chiral séparable un énantiomère de l'acide di-0,0'-p-toluyl-tartrique ou de l'acide di-0,0'-benzoyl-tartrique.

4. Procédé selon la revendication 2, caractérisé en ce que l'on fait réagir le racémate de formule I avec l'acide (+)-di-0,0'-p-toluyl-D-tartrique ou l'acide (+)-di-0,0'-benzoyl-D-tartrique servant d'acide séparable et l'on sépare de la liqueur mère le S-céliprolol-di-0,0'-p-toluyl-D-tartrate ou le S-céliprolol-di-0,0'-benzoyl-D-tartrate qui précipite lors de la cristallisation fractionnée.

5. Procédé selon l'une quelconque des revendications 2 à 4, caractérisé en ce que, lors de la séparation racémique, la formation de sel avec l'acide chiral et la cristallisation fractionnée sont mis en œuvre dans le méthanol, l'éthanol, ou un mélange de ces alcools et d'eau.

6. Procédé selon l'une quelconque des revendications 2 à 5, caractérisé en ce que l'on transforme, à l'aide d'un acide minéral, le sel diastéréoisomère, obtenu après séparation racémique à partir de S(—)-céliprolol et d'acide séparable, directement en sel d'addition d'acide pharmaceutiquement acceptable, cette transformation étant effectuée dans un solvant dans lequel l'acide séparable organique libéré est

facilement soluble et en ce que le sel minéral obtenu après transformation est difficilement soluble dans le S(—)-céliprolol et précipite.

7. Procédé selon l'une quelconque des revendications 2 à 6, caractérisé en ce que, pour la préparation de sels d'addition d'acide pharmaceutiquement acceptables, on emploie l'acide chlorhydrique.

8. Procédé selon l'une quelconque des revendications 2 à 7, caractérisé en ce que l'on effectue ladite transformation directe des sels obtenus après séparation racémique, à partir de S(—)-céliprolol et d'acide séparable, à l'aide d'acides minéraux solubles dans un solvant consistant en acétone.

9. Composition pharmaceutique caractérisée en ce qu'elle est constituée du dérivé selon la revendication 1, ou de ses sels et des liants, supports et/ou adjuvants pharmaceutiquement acceptables habituels.

**Revendications** (pour l'Etat contractant AT)

1. Procédé de préparation de S(—)-3-[3-acétyl-4-(3-tertio-butylamino-2-hydroxypropoxy)phényl]-1,1-diéthylurée (S(—)-céliprolol) et ses sels d'addition d'acide pharmaceutiquement acceptables, caractérisé en ce que

a) l'on fait réagir le racémate de formule

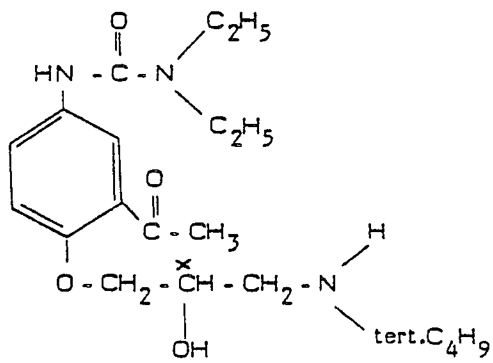

(I)

sous forme de base, avec un énantiomère d'un acide chiral séparable dans un solvant organique approprié, l'on sépare par cristallisation fractionnée le mélange des deux sels diastéréoisomères ainsi obtenu et, après séparation, l'on retransforme chaque sel diastéréoisomère qui contient l'énantiomère lévorotatoire du dérivé de formule I en configuration-S sous forme de base libre ou sous forme d'un sel d'additon d'acide pharmaceutiquement acceptable, et l'on récupère l'acide séparable, ou bien

b) l'on fait réagir la S(+)-3[3-acétyl-4-(2,3-époxy-propoxy)phényl]-1,1-diéthylurée de formule

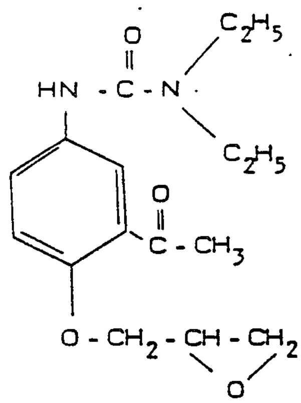

(II)

avec la tertiobutylamine, en présence d'eau, à la température ambiante ou sous chauffage modéré à 40 °c maximum et l'on transforme, le cas échéant, l'énantiomère lévorotatoire de formule I ainsi obtenu en un sel d'addition d'acide pharmaceutiquement acceptable.

2. Procédé selon la revendication 1, caractérisé en ce que, pour la séparation racémique, on emploie comme acide chiral séparable un énantiomère de l'acide di-0,0'-p-toluyl-tartrique ou de l'acide di-0,0'-benzoyl-tartrique.

3. Procédé selon l'une des revendications 1 ou 2, caractérisé en ce que l'on transforme le racémate de formule I par action d'acide séparable consistant en acide (+)-di-0,0'-p-toluyl-D-tartrique ou acide (+)-di-0,0'-benzoyl-D-tartrique de sorte que se sépare de la liqueur mère par cristallisation fractionnée un

précipité consistant en D-tartrate de S-céliprolol-di-0-0′-p-toluoyl ou D-tartrate de S-céliprolol-di-0-0′-p-benzoyl.

4. Procédé selon l'une quelconque des revendications 1 à 3, caractérisé en ce que, lors de la séparation racémique, la formation de sel avec l'acide chiral et la cristallisation fractionnée sont mis en œuvre dans le méthanol, l'éthanol, ou un mélange de ces alcools et d'eau.

5. Procédé selon l'une quelconque des revendications 1 à 4, caractérisé en ce que l'on transforme, à l'aide d'un acide minéral, le sel diastéréoisomère, obtenu après séparation racémique à partir de S(—)-céliprolol et d'acide séparable, directement en sel d'addition d'acide pharmaceutiquement acceptable, cette transformation étant effectuée dans un solvant dans lequel l'acide séparable organique libéré est facilement soluble et en ce que le sel minéral obtenu après transformation est difficilement soluble dans le S(—)-céliprolol et précipite.

6. Procédé selon l'une quelconque des revendications 1 à 7, caractérisé en ce que, pour la préparation de sels d'addition d'acide pharmaceutiquement acceptables, on emploie l'acide chlorhydrique.

7. Procédé selon l'une quelconque des revendications 1 à 6, caractérisé en ce que l'on effectue ladite transformation directe des sels obtenus après séparation racémique, à partir de S(—)-céliprolol et d'acide séparable, à l'aide d'acides minéraux solubles dans un solvant consistant en acétone.